# EUROPEAN PATENT SPECIFICATION

(11) **EP 0 379 549 B1**
(45) Date of publication and mention of the grant of the patent: **27.12.1995**
(21) Application number: 89907603.8
(22) Date of filing: 21.06.1989
(51) Int. Cl.: C07K 1/00, C07K 14/435, G01N 27/26, G01N 33/567

(54) **ENDOTOXIN BINDING PROTEIN AND USES THEREOF**
ENDOTOXIN-BINDEPROTEIN UND DESSEN VERWENDUNG
PROTEINE DE LIAISON D'ENDOTOXINES ET SES UTILISATIONS

(30) Priority: 23.06.1988 US 210575
(43) Date of publication of application: 01.08.1990
(60) Divisional application: 95201582.4
(73) Proprietor: ASSOCIATES OF CAPE COD, INC., Falmouth Massachusetts 02543 (US)
(72) Inventor: WAINWRIGHT, Norman, R., Falmouth, MA 02540 (US); NOVITSKY, Thomas, J., Falmouth, MA 02540 (US)
(74) Representative: Sheard, Andrew Gregory
(86) International application number: US8902665
(87) International publication number: WO8912644

(56) References cited:
- US-A- 4 713 347
- US-A- 4 758 655
- US-A- 4 780 529
- THE JOURNAL OF BIOLOGICAL CHEMISTRY, vol. 255, no. 12, 25th June 1980, pages 5586-5590; S.-M. LIANG et al.: "Studies on limulus amoebocyte lysate. III. Purification of an endotoxin-binding protein from limulus amoebocyte membranes"
- JOURNAL OF BIOCHEMISTRY, vol. 101, no. 6, 1987, pages 1321-1330; T. MUTA et al. : "Primary structure of anti-lipopolysaccharide factor from American horseshoe crab, Limulus polyphemus"
- THE JOURNAL OF BIOLOGICAL CHEMISTRY, vol. 261, no. 16, 5th June 1986, pages 7357-7365, The American Society of Biological Chemists, Inc., Baltimore, MD, US; J. AKETAGAWA et al.: "Primary structure of limulus anticoagulant anti- lipopolysaccharide factor"
- Advances in Biochemical Engineering/-Biotechnology, Springer Verlag, N.Y., 1983, pp. 120-123, Flaschel
- The Journal of Biological Chemistry, vol. 256, 1981, pp. 4968-4972, Liang

## Description

### Technical Field

The present invention is directed to pharmaceutical compositions and uses of an endotoxin binding protein isolated from a horseshoe crab. The invention covers, inter alia, pharmaceutical compositions and pharmaceutical uses of the protein, a method of purifying the protein, and an assay for endotoxin based on this protein.

### Background Art

Endotoxins are high molecular weight complexes, associated with the outer membrane of gram-negative bacteria, that produce pyrogenic reactions upon intravenous administration. Endotoxin is shed from living bacteria and is also released into the environment when bacteria die and decompose. Since gram-negative bacteria are found in great numbers in air, water, and soil, bacterial endotoxin commonly contaminates raw materials and processing equipment used in the manufacturing of, for example, pharmaceuticals.

Bacterial endotoxin is a complex consisting of lipid, carbohydrate and protein. It is characterized by an overall negative charge, heat stability and high molecular weight. Highly purified endotoxin does not contain protein, and is a lipopolysaccharide (LPS). Depyrogenation can generally be achieved by inactivating or removing endotoxin, depending upon the physicochemical nature of the LPS. LPS consists of three distinct chemical regions, lipid A, which is the innermost region, an intermediate core polysaccharide, and an outermost O-specific polysaccharide side chain which is responsible for an endotoxin's particular immunospecificity.

Bacterial endotoxins are known to have profound biological effects in animals and humans, and to cause severe medical problems when present. Symptoms include induction of high fever, activation of complement, and hypotension. It is critical to avoid endotoxin contamination in any pharmaceutical product or medical device which comes into contact with body fluids. High endotoxin levels in sera due to bacterial diseases, such as septicemia, are not easily treated. Antibiotic treatment of the infection only kills the bacteria, leaving the endotoxin from their cell walls free to cause fever.

The horseshoe crab Limulus polyphemus is particularly sensitive to endotoxin. The cells from their hemolymph (amebocytes) undergo a complex series of biochemical reactions resulting in clot formation, analogous to mammalian blood coagulation. This phenomenon has been exploited in the form of bioassays sensitive to very low endotoxin levels. Currently, a bioassay of this type is the method of choice for monitoring pharmaceutical manufacturing and is termed Limulus Amebocyte Lysate (LAL). See U.S. Patents 4,276,050, 4,273,557, 4,221,866, 4,201,865, 4,038,147, 3,944,391 and 3,915,805, each of which is incorporated herein by reference.

It has long been observed that once endotoxin interacts with LAL the toxin is not recoverable from the clot. See Nachum et al, Journal of Invertebrate Pathology, 32:51-58 (1978). This observation lead investigators to postulate two alternative explanations. Either the endotoxin is enzymatically degraded during clot formation or it is bound by some factor causing it to lose toxicity. The present inventors initiated experiments to extract the endotoxin inactivating factor from the LAL.

Other research groups have experimented with endotoxin binding proteins, also referred to as anti-LPS factor. To the inventor's knowledge, the following publications resulting from work in this area are the most relevant to this invention:
Tanaka et al, Biochem. Biophys. Res. Comm. 105, 717-723 (1982),
Iwanaga et al, International Symposium on Pyrogen, 84-84 (June 23-26, 1987),
Aketagawa et al, J. Biol. Chem. 261, 7354-7365 (1986),
Hao, U.S. Patent 4,677,194 (June 30, 1987), and
Nachum et al, J. Inv. Path. 32, 51-58 (1978).

Tanaka et al, Iwanaga et al, and Aketagawa et al each conducted research on an anti-LPS factor or endotoxin binding protein isolated from a horseshoe crab system. Based on experimental work done in the inventors' laboratory, it appears that a protein involved in the present invention is the same as that isolated by Iwanaga et al and Tanaka et al. However, these publications do not say anything about pharmaceutical utility of the endotoxin binding protein, and it is difficult to predict in vivo activity based on in vitro experimentation. In fact, neither of these references suggests a practical utility for the anti-LPS factor, and in view of the unpredictable nature of in vivo activity, it has previously not been appreciated that the endotoxin binding protein could be used in a pharmaceutical composition. Furthermore, none of the references disclose the use of the endotoxin binding protein for an endotoxin assay, as disclosed in the present invention. It is notable that the present inventors have also discovered certain endotoxin binding protein variants which have amino acid structures that are different from the anti-LPS factor disclosed in the above-described publications.

### Disclosure of the Invention

Accordingly, it is an object of the present invention to provide pharmaceutical compositions containing therein an endotoxin binding protein isolated from a horseshoe crab, which compositions are capable of binding endotoxin in vivo.

It is yet another object of the present invention to provide a method for reducing endotoxin concentration in vivo.

It is yet another object of the present invention to provide a method for purifying an endotoxin binding protein from a horseshoe crab.

These and other objects of the present invention which will hereinafter become more readily apparent. have been provided by purifying an endotoxin binding protein from the horseshoe crab Limulus polyphemus, and discovering that it has the capability of binding to endotoxin in vivo. The present inventors have also discovered that there are certain structural variants of the Limulus polyphemus endotoxin binding protein, and it is expected that these materials will also possess endotoxin binding ability such that they may be used in the other aspects of the present invention as well.

### Brief Description of the Drawings

A more complete appreciation of the invention and many of the attendant advantages thereof will be readily obtained as the same becomes better understood by reference to the following detailed description when considered in connection with the accompanying drawings, wherein:

FIGURE 1 shows cation exchange chromatographic purification of Limulus endotoxin binding protein using CM-Sepharose® resin. Peak 1: flow through, Peak 2: 0.25M NaCl elution, Peak 3: 1 M NaCl elution.

FIGURE 2 shows reversed phase chromatographic purification of Peak 3 from Fig. 1. Peak A: 25% isopropanol (IPA) elution, Peak B: 35% IPA elution, Peak C: 50% IPA elution, Peak D: column wash with 100% IPA.

FIGURE 3 is a plot of apparent endotoxin concentration (measured by LAL) versus protein concentration. Such a plot can be used to assess protein specific endotoxin inactivating activity. Specific activity is expressed as the amount of protein needed to achieve 50% reduction of endotoxin in the assay system (5 nanograms of endotoxin per 100 micro-liters). For convenience, units are expressed as micrograms⁻¹ protein x 10⁵ needed to reduce 5 nanograms/100 microliters of standard endotoxin by 50%.

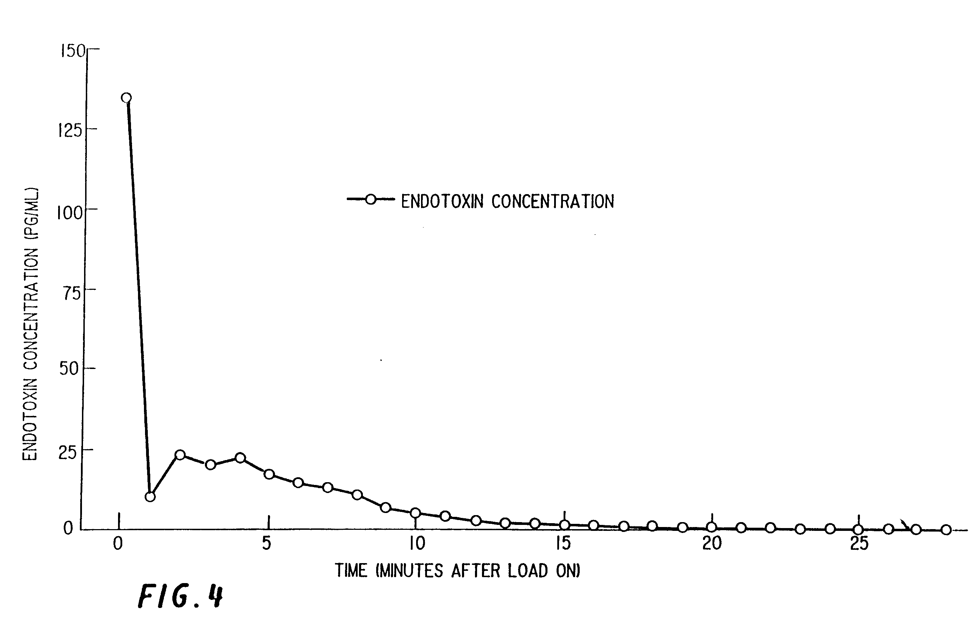

FIGURE 4 shows depyrogenation by affinity chromatography with endotoxin binding protein immobilized to a solid support.

FIGURE 5 shows fluorescence excitation and emission spectra of Limulus endotoxin binding protein.

FIGURE 6 shows successive decreases in the emission spectra of Limulus endotoxin binding protein upon successive additions of endotoxin.

FIGURE 7 shows lack of successive decreases in the emission spectra of human serum albumin upon successive additions of endotoxin (negative control).

FIGURE 8 shows lack of successive decreases in the emission spectra of peak B, FIGURE 2 upon successive additions of endotoxin (negative control).

FIGURE 9 shows a fluorescence titration of Limulus endotoxin binding protein at pH 3.86. K_{D} = 2.41 micromolar.

FIGURE 10 shows fluorescence titration of Limulus endotoxin binding protein at pH 6.91. K_{D} = 0.51 micromolar.

FIGURE 11 shows fluorescence titration of Limulus endotoxin binding protein at pH 8.80. K_{D} = 2.40 micromolar.

FIGURE 12 shows the effect of pH on dissociation constant of Limulus endotoxin binding protein.

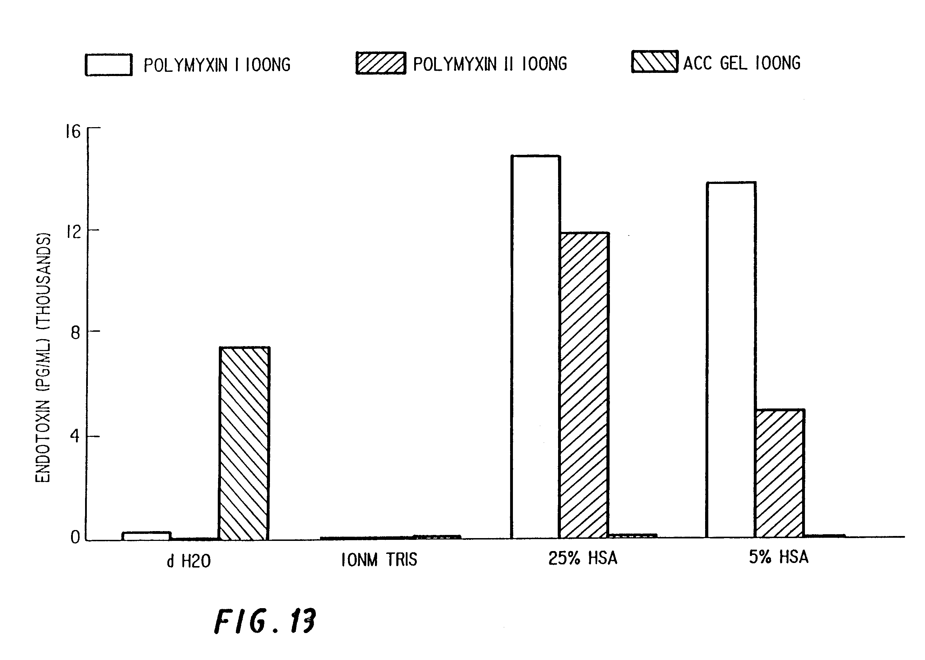

FIGURE 13 is a comparison of immobilized Limulus endotoxin binding protein compared to two immobilized polymyxin resins in proteinaceous and non-proteinaceous solutions.

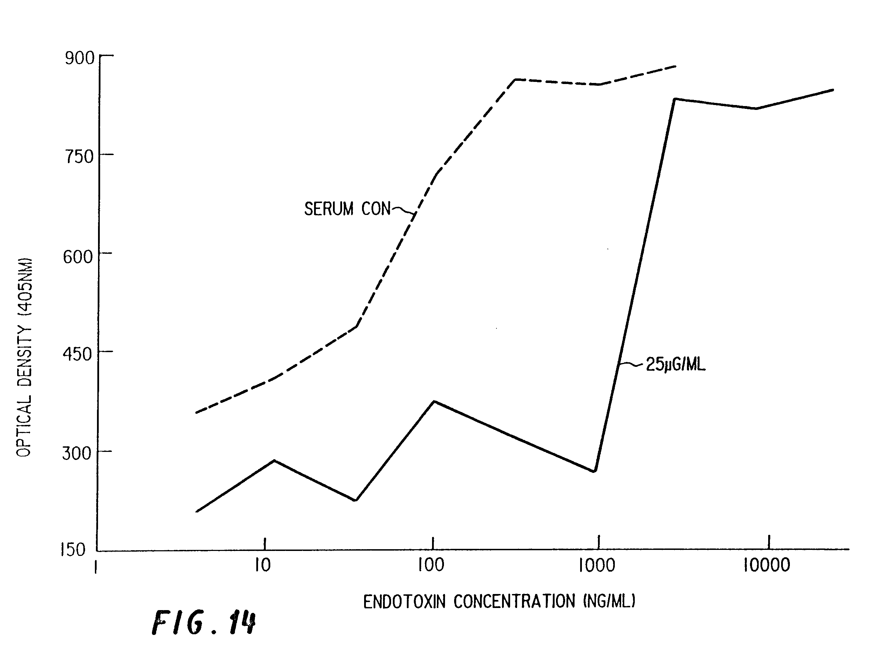

FIGURE 14. Demonstration of activity of Limulus endotoxin binding protein in the presence of total human serum. The dashed line represents serum alone as a control (68 nanograms of endotoxin per ml inactivated). The solid line represents serum plus 25 micrograms/ml added endotoxin binding protein (1600 nanograms endotoxin per ml inactivated).

### Best Mode For Carrying Out the Invention

The endotoxin binding protein of the present invention may be isolated from any horseshoe crab. For example, any of the four known species of horseshoe crabs could be used. These species are:
Limulus polyphemus
Tachypleus gigas
Tachypleus tridentatus
Carcinoscorpius rotundicauda
Especially preferred among these is Limulus polyphemus, the horseshoe crab which is found along the North American coast. The endotoxin binding protein may be isolated by a procedure which is summarized hereinbelow. The procedure is illustrated for the Limulus horseshoe crab, but these procedures may be applied to any known horseshoe crab, as recited herein.

It was surprisingly found that the cellular debris produced during lysate production from Limulus amebocytes contains significant amounts of the endotoxin binding protein. The cellular debris was found to have even more activity than lysate itself. By "cell debris" is meant the insoluble material remaining when Limulus amebocytes are lysed by hypotonic shock. It includes nuclei, cell debris and some lysate proteins. The majority is insoluble material. Hypotonic shock may be accomplished by treating the Limulus amebocytes with endotoxin-free distilled water, preferably at 4°C, with shaking (e.g., for 12 h). The mixture is centrifuged, separating the lysate (soluble supernatant) from the cell debris.

The next step is to extract from the cell debris the Limulus amebocyte binding protein. Many solvents have been tested, including water, several alcohols (e.g. methanol, ethanol, isopropanol, and butanol), acetone, chloroform, acetonitrile, acids (e.g. HCl and H₂SO₄), bases (e.g. NaOH and ethanolamine), salts (e.g. NaCl), and detergents (e.g. Tween, triton X-100, and SDS). The best results were obtained with denaturants such as urea and guanidine hydrochloride. Surprisingly, six molar solutions of denaturants were effective in extracting the protein without affecting biological activity. This concentration of denaturant would be expected to inactivate most proteins. Thus, the first step in the purification procedure herein is extraction of cellular debris from Limulus amebocyte lysate with a denaturant to produce an extract. The concentration of the denaturant can range from 1 M to 10 M. A more preferred range would be 3 M to 8 M. A most preferred concentration is around 6 molar. Urea is the preferred denaturant, because it can be made free of endotoxin by ultrafiltration and it is not readily contaminated by endotoxin containing bacteria.

Preferably, prior to loading the extract onto a cation exchange column, an ultrafiltration step is performed. This step is also accomplished using urea or another denaturant as described above for extraction. In particular, the extract from the cell debris is crudely filtered with a filter aid such as diatomaceous earth (e.g. Celite, Manville Corp.) or one of the cationic or anionic polymeric particles in colloidal suspension (e.g. Biocryl, Supelco division of Rohm and Haas Corp.), then passed through an ultrafiltration membrane. Ultrafiltration membranes may be used in any known form (e.g., plain film, hollow fiber, tubular and spiral) and may be made of any material. Preferred materials are polysulfone, polyvinylidene fluoride, polyacrylonitrile, nylon, or cellulose. Most preferred is a polysulfone, flat or hollow fiber type ultrafiltration membrane. The preferred molecular weight cut-off of the ultrafiltration membrane is 20,000 daltons to 50,000 daltons as can be commercially obtained from Millipore, Filtron or other membrane filter manufacturers. The most preferred membrane has a 30,000 dalton cut-off. The Limulus endotoxin binding protein is now in the filtrate at a very low concentration. The filtrate from the first membrane is concentrated using a second ultrafiltration membrane having a molecular weight cut-off of 5,000 to 10,000 daltons, preferably an 8,000 dalton cut-off membrane. The second ultrafiltration membrane may be the same or different material as the first ultrafiltration membrane, and is preferably made of polysulfone. The principle of operation is the same as the 30,000 cut-off membrane, however, the endotoxin binding protein is now in the retentate with all other proteins greater than e.g. 8,000 daltons.

After the above step, the retentate is subjected to cation exchange chromatography using cellulose, cross-linked agarose or hydrophilic vinyl polymer resins derivatized with carboxymethyl (CM), Sulphopropyl (SP), Sulphonate (S), or other anionic group. These can be obtained from several manufacturers including Pharmacia (Sephadex®, Sepharose®), Tosoh Corp. (Toyopearl®) or BioRad (Cellex®, Bio-Gel). The most preferred resin is CM-Sepharose®. The pH of loading and elution buffers can range from 4 to 8, with the most preferred range of pH 5 to 5.5. In this step also, urea (or another denaturant as described above for extraction) is an important constituent. All column elution buffers must contain urea (at least about 3 molar) to elute the endotoxin binding protein cleanly from the column.

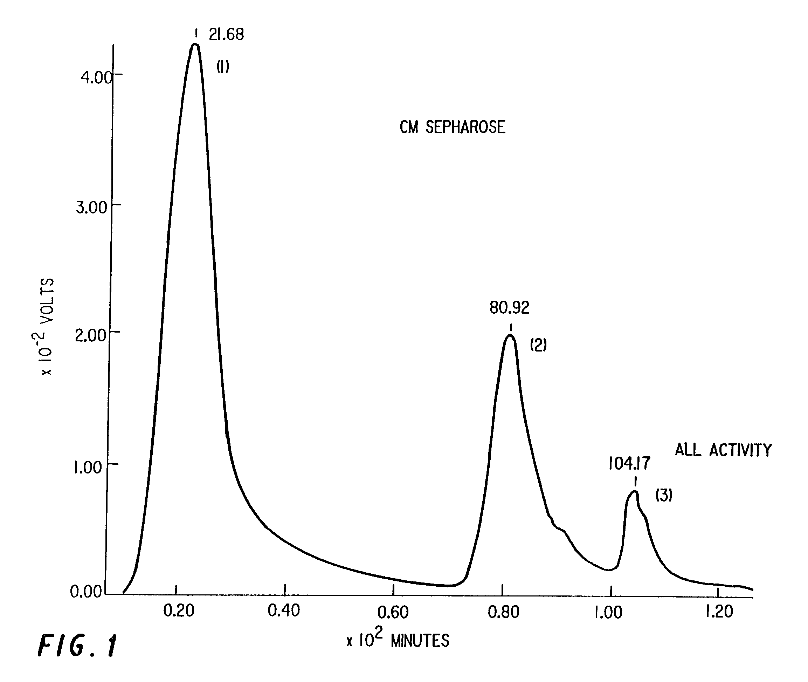

The elution from the column is accomplished by a step gradient of a salt such as ammonium chloride, potassium chloride or sodium chloride. Sodium chloride is preferred. FIGURE 1 shows the results of the CM Sepharose® chromatographic step. The preferred concentration of urea in the eluent used in this step is 1 M to 6 M. A preferred concentration of urea in this step is 2 M to 4 M. A most preferred concentration is 2.5 M to 3.5 M. When sodium chloride is used in the eluant, biological activity elutes at a concentration of sodium chloride of from around 0.5 to 1 molar.

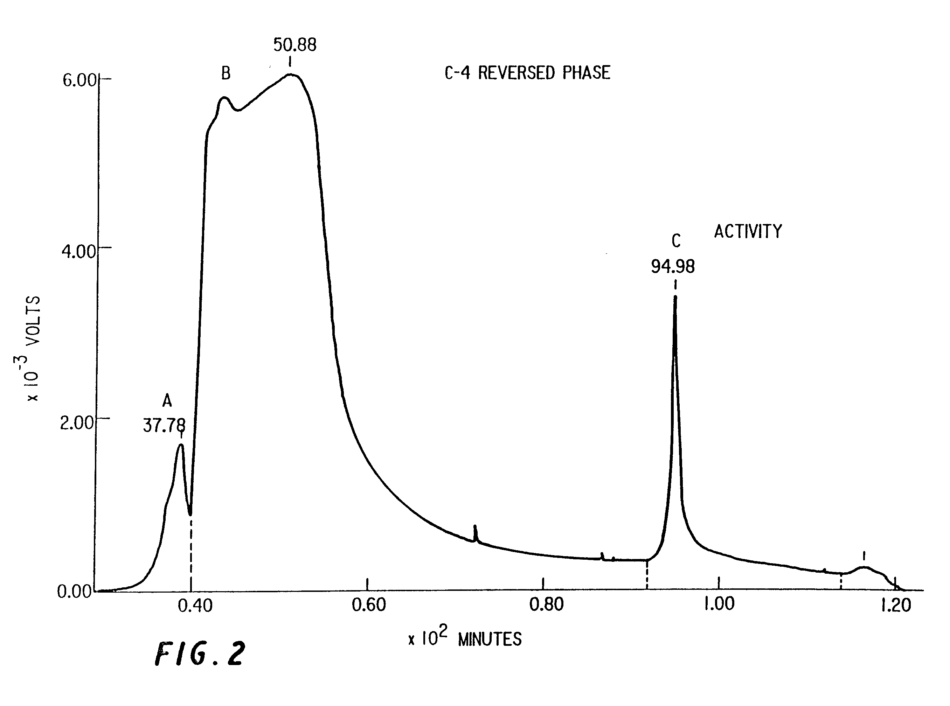

After the cation exchange step, the salt-eluted peak of activity is applied to a reversed-phase column. The preferred method employs a resin having 4, 8, or 18 carbon chains (C-4, C-8 or C-18, respectively). The most preferred method employs a C-4 resin commercially available from such resin manufacturers as Vydac, Waters, Tosoh Corp., etc. The protein is eluted by a step gradient of, for example, isopropanol and trifluoroacetic acid (TFA). The concentration of trifluoroacetic acid is ideally 0.2%, but can range from 0 to 0.4%, preferably 0.15 to 0.25%. FIGURE 2 shows the location of the activity during treatment on the reversed-phase column. The reversed-phase column step effects desalting and further purifies the material to virtual homogeneity. The Limulus protein is remarkably stable to the organic solvents and low pH of this column. The protein is stable over a pH range of 1-3 in the presence of TFA. The material may now be lyophilized. The purified Limulus protein has an isoelectric pH (pI), which is greater than 10, indicating a very basic protein. The molecular weight of the protein as determined by SDS-PAGE is 12,500 ± 100. The first three N-terminal amino acids of this purified material are
Asp-Gly-Ile
It should be noted that Watson and Sullivan, U.S. Patent 4,107,077, teaches that there is an increase in sensitivity of the lysate by organic extraction with chloroform. It appeared logical that a protein which inactivated endotoxin would appear as an inhibitor in the endotoxin assay. Thus, it was hoped that the protein was the inhibitor which is extracted into chloroform and could be recovered from that extract. However, surprisingly, this was not successful. Since the protein is fairly hydrophobic, it may be in the organic extract, but denatured in some way.

The majority of the purified material had an amino acid sequence which appears to be identical to a protein from Limulus that was isolated by Tanaka et al. The material was subsequently purified by Iwanaga et al, as reported in the International Symposium on Pyrogen, held June 23,-26, 1987 in China. However, in the latter case, a different purification procedure was employed.

In addition to a protein having an apparently identical amino acid sequence to that reported in Iwanaga et al, some related proteins were also purified, which have different amino acid sequences. These variant proteins have not previously been described. In a first protein, on the N-terminal thereof, a serine rather than an aspartic acid residue is located. Furthermore, an asparagine is located in the second position from the N-terminal rather than a glycine as reported in Iwanaga et al. Therefore, a protein having an initial amino acid sequence of
Ser-Asn-Ile-Trp-Thr-
is also part of the present invention. Other possible protein derivatives are those beginning with
Asp-Asn-
Ser-Gly-, and
Ser-Asn-,
and also a protein having an N-terminal asparagine with one less amino acid than the natural sequence. Each of these variant proteins is also part of the present invention, and they may be used in each aspect of the invention described hereinbelow. They will be referred to as endotoxin binding protein variants (or variant proteins for short), as distinguished from the natural sequence endotoxin binding protein, which is the protein having the amino acid sequence reported in Iwanaga et al.

The variant proteins were not separated from each other. Rather, upon sequencing the most purified samples, some positions were uniformly one amino acid, while other positions showed major variation. This is interpreted as a mixture of proteins showing microheterogeneity. It is consistent with the existence of a gene family coding for these proteins whose individual gene sequences are very homologous, but not identical. It remains to be seen if their individual specific activities vary significantly.

The present invention also encompasses gene sequences which encode the polypeptides of the present invention, vectors containing these gene sequences, and microorganisms such as E. coli, yeast, etc., transformed with the vectors. The transformed microorganisms can be used to produce large quantities of the polypeptide materials, including the variant proteins described above. The genes of this invention may be altered so as to maximize codon expression in a given host.

### Procedure for Assaying for Endotoxin Binding Activity

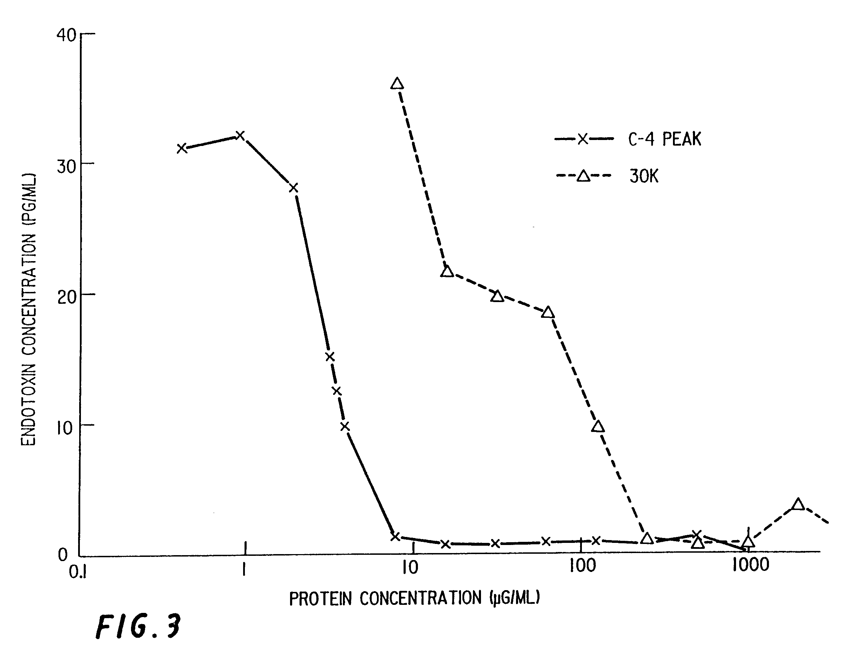

To follow the purification of the endotoxin binding protein during the above purification steps, assays can be performed in solution. One publication describing typical assays for LR₅₀ values is Novitsky et al, J. Clin. Microbiol. pp. 211-216 (1985). A specific procedure follows:
A 96-well microtiter plate is used. Protein fractions are serially diluted across the top row and mixed with a standard endotoxin solution. There is another series of dilutions done on all samples to get within range of the assay, but basically, the endotoxin recovered in the well after mixing with a suspected endotoxin inactivating protein is measured. This is compared to negative controls and an endotoxin standard curve. It is found that the added endotoxin is inactivated at the high protein concentrations. As the protein is diluted across the plate, a point is reached where it is too dilute to inactivate the added endotoxin. This dilution is a measure of the protein specific activity. FIGURE 3 represents a graphical form of such experiments. In FIGURE 3, the dotted curve represents the crude extract after ultrafiltration, and the solid line is the most highly purified fraction from the reversed-phase column.

### In Vivo Activity of the Endotoxin Binding Protein of the Present Invention

The endotoxin binding protein of the present invention (and variants thereof) may be incubated with endotoxin, and then administered to animals, and the bound endotoxin is not found to cause any pyrogenicity in vivo. The details of the endotoxin test are presented hereinbelow in the examples section.

Surprisingly, and even less predictably, the present endotoxin binding protein may be administered to an animal after the animal has already been exposed to endotoxin, and the endotoxin binding protein can reverse the effects of free endotoxin in vivo. Moreover, the endotoxin binding protein may be administered to an animal before contact with endotoxin by the animal, and the endotoxin binding protein will exert a protective effect against the effects of endotoxin. Accordingly, the endotoxin binding protein of the present invention may be formulated into a pharmaceutical composition for treating an animal in vivo, so as to exert a therapeutic effect if endotoxin is present in the animal, or to exert a protective or preventive effect, if the animal should come into contact with endotoxin later. Details on the experimental tests showing the in vivo effects of the endotoxin binding protein of the present invention are also presented in the examples section hereinbelow.

It should be noted that in vivo activity of this type would have been unpredictable based on the bare disclosure of the in vitro endotoxin binding capability of this protein. According to Nachum (described above), there were two possibilities for the removal of endotoxin by lysate proteins, binding or enzymatic cleavage of endotoxin. The inventors looked for degradation products and found none (specifically, free fatty acids that could have been released by esterases). Subsequent work has found binding to be the mechanism. It is more expected that the endotoxin would be inactivated in vivo by an enzymatic action - the molecule would be structurally different. However, by only binding, one would assume the entire endotoxin structure would still be present and available to trigger the normal biological response to the toxin. The noted inactivation in vitro might easily have been an artifact of an artificial assay system - perhaps a conformational change in the bound endotoxin not reacting with the LAL reagent, or it is also possible that the Limulus endotoxin binding protein was inhibiting the clotting reaction by itself (since it is present in lysate, this possiblity is hard to rule out without the in vivo results).

Although it might be possible for the endotoxin binding protein of the present invention to be administered in vivo without any additives thereto, it is preferably mixed with a carrier, and if necessary, other adjuvants.

By the term "carrier" as used herein is meant a synthetic or natural, inorganic or organic substance which is added to the endotoxin binding protein of the present invention to assist the active ingredient in reaching the location to be treated therewith and to facilitate storage, transportation and handling of the active ingredient.

Among suitable liquid carriers, there may be included aromatic hydrocarbons such as benzene, toluene, xylene, cumene, etc., paraffinic hydrocarbons such as mineral oil and the like, halogenated hydrocarbons such as carbon tetrachloride, chloroform, dichloroethane and the like, ketones such as acetone, methyl ethyl ketone, etc., ethers such as dioxane, tetrahydrofuran and the like, alcohols such as methanol, propanol, ethylene glycol and the like, dimethyl formamide, dimethylsulfoxide, water, etc. Mixtures of any number of liquid carriers are also envisioned. Upon dissolution of lyophilized active ingredient with unbuffered pyrogen free distilled water or saline or phosphate buffered saline (pH 6.5 - 7.5), the protein is not completely soluble. In order to avoid undesirable physiological side-effects which might result from such suspensions, the pH may be adjusted to slightly alkaline pH (pH 8-9) at which the material becomes water clear. For this reason, the most preferred liquid carrier is pyrogen free distilled water or saline adjusted to an alkaline pH.

In order to enhance the effectiveness of the compound according to this invention, it is possible to use such adjuvants as given below either singly or in combination in accordance with the purpose of each application thereof while taking into consideration the form of their preparation and their field of application.

Namely, exemplary adjuvants may include anionic surfactants such as alkyl sulfates, aryl sulfonates, succinates, polyethylene glycol alkyl aryl ether sulfates, and the like, cationic surfactants such as alkylamines, polyoxyethylene alkylamines, etc., non-ionic surfactants such as polyoxyethylene glycol ethers, polyoxyethylene glycol esters, polyol esters and the like, and amphoteric surfactants. Encapsulation or microencapsulation of the active ingredient in liposome vesicles is also within the scope of this invention.

Examples of stabilizers, thickeners, lubricants and the like are isopropyl hydrogen-phosphate, calcium stearate, wax, casein, sodium alginate, serum albumin, other blood proteins, methylcellulose, carboxymethylcellulose, gum arabic, etc. It should be kept in mind that these ingredients are not limited to the recited examples.

The active materials according to the present invention can be administered by any route, for example, orally, parenterally, intravenously, intradermally, subcutaneously, or topically, in liquid or solid form. Preferably, the route of administration is intravenously.

The solutions or suspensions may also include the following components: a sterile diluent such as water for injection, saline solution, oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple base vials made of glass or plastic.

While dosage values will vary with the specific severity of the disease condition to be alleviated, good results are achieved when the compounds described herein are administered to a subject requiring such treatment as an effective oral, parenteral or intravenous dose of from 100 to 10,000 units per nanogram of measured endotoxin per day per patient. A particularly preferred effective amount is about 1000 to 5000 units per nanogram of measured endotoxin per day per patient. It is to be understood, however, that for any particular subject, specific dosage regimens should be adjusted to the individual need and the professional judgment of the person administering or supervising the administration of the aforesaid compound. It is to be further understood that the dosages set forth herein are exemplary only and they do not limit the scope or practice of the invention. The dosages may be administered at once, or may be divided into a number of smaller doses to be administered at varying intervals of time.

Specific diseases which could be treated include septicemia, toxic shock, and any other condition, especially gram-negative bacterial infections, which is accompanied by an increase in in vivo endotoxin content. Other examples are endotoxin-related arthritis, gonorrhea, periodontal disease, spinal meningitis, and infections of amniotic fluid.

Although this invention and its preferred embodiments are primarily addressed to use in humans, veterinary use is also encompassed by the invention. In this regard, the active ingredient may be administered to reduce or prevent the pyrogenic or other ill effects of endotoxin in vivo in, for example, dogs, cats, horses, cattle, sheep, and rabbits. Administration to laboratory animals, such as mice and rats, in order to prevent or reduce effects of endotoxin is also contemplated.

Although polyclonal and monoclonal antibodies to endotoxin have also been developed for this purpose and are the subject of ongoing development, the endotoxin binding protein of the present invention has advantages over antibodies which make it a preferred candidate for a therapeutic. The binding constant is very high and its low molecular weight may be less antigenic. In a recent paper, Greisman and Johnston, J. Infect. Disease 157:54-64 (1988), it has been shown that at least one anti-serum against endotoxin is ineffective in reducing the effects of endotoxin in vivo. In contrast, the in vivo experiments in the present invention have shown that the endotoxin binding protein may inactivate endotoxin in both rabbit and human serum.

### Removal of Endotoxin From Solutions

Endotoxin contamination of pharmaceuticals is a problem toward which many removal protocols have been directed. Many of these are summarized in Biopharm, April, 1988, pages 22-29, and the references cited therein. They all fall short when the contaminated material is or contains a high molecular weight substance, such as protein. Most of the existing technology for endotoxin removal (ultrafiltration, ion exchange, affinity chromatography) does not separate protein and endotoxin. The present invention involves using the endotoxin binding protein (e.g. from Limulus) as an immobilized affinity ligand to achieve this separation. The preferred solid support for covalently coupling the endotoxin binding protein is cellulose, agarose or other hydrophilic polymer derivatized to contain carboxyl, hydroxyl, amino, epoxide or other chemically reactive group to which proteins may be covalently attached using well known coupling chemistry. These methods include using water soluble carbodiimide or carbonyldiimidazole, glutaraldehyde, etc. The most preferred support is the hydrophilic vinyl polymer in pellicular or membrane form containing carboxymethyl groups activated with water-soluble carbodiimide. The inventors have attached the binding protein covalently to chromatographic beads and the protein retains its biological activity. Furthermore, by mixing the beads with endotoxin contaminated proteins, the solution could be detoxified. This was done by mixing the beads in batch mode in test tubes, or by packing a column with the beads and passing the solutions over it.

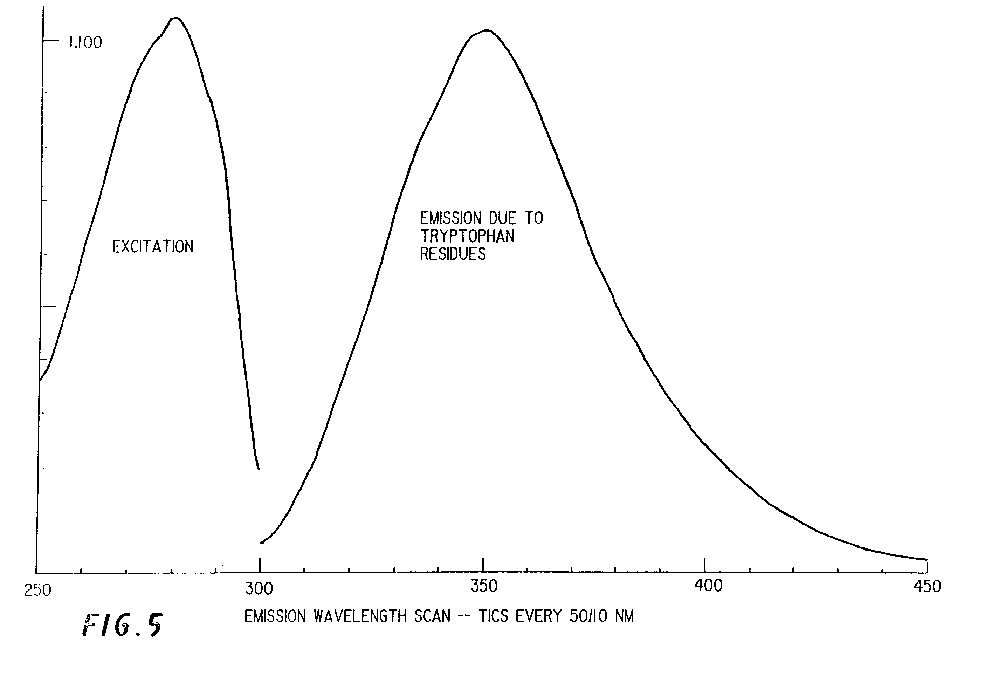

The protein could also be covalently attached to filter membranes for the same purpose. FIGURE 5 shows an experiment with such a filter. A 40 ml solution containing approximately 130 pg/ml endotoxin was recirculated through a filter containing the binding protein. There was an initial large drop in the toxin concentration that was further reduced as the material recirculated.

The present inventors further conducted a direct comparison of too immobilized polymyxin resins compared to the present ligand on similar gel particles. The experiment took 100 microliter gel volumes or each in a microcentrifuge tube and added 100 nanograms of E. coli endotoxin in 1 ml of various solutions to each. The tubes were mixed end over end for one hour. In 10 mM Tris, all three performed equally well. In 25% human serum albumin (HSA) and 5% HSA, the present endotoxin binding protein was able to remove significantly more endotoxin. Interestingly, in distilled water, polymyxin was superior. Thus, the present endotoxin binding protein exhibits surprisingly superior performance in protein-containing solutions. Other possible proteins contaminated by endotoxin are HSA, interferon, interleukins, growth factors, hormones, proteases, TPA, TNF, monoclonals, EGF, insulin, and erythropoetin.

### Extracorporeal Treatment of Septic Shock

Because of the ability of the endotoxin protein to function in protein solutions and human serum, an affinity membrane or hollow fiber may be constructed for the purpose of removing endotoxin from the blood of patients extracorporeally. In such a mode, similar to kidney dialysis, blood containing elevated concentrations of endotoxin from a variety of potential clinical conditions is circulated through an affinity device such that the serum is brought into direct contact with endotoxin binding protein covalently bound to the membrane. Since the amount of endotoxin binding protein being released into the general circulation is much reduced from an intravenous application, potential side effects are minimized.

### Assay for Binding Affinity

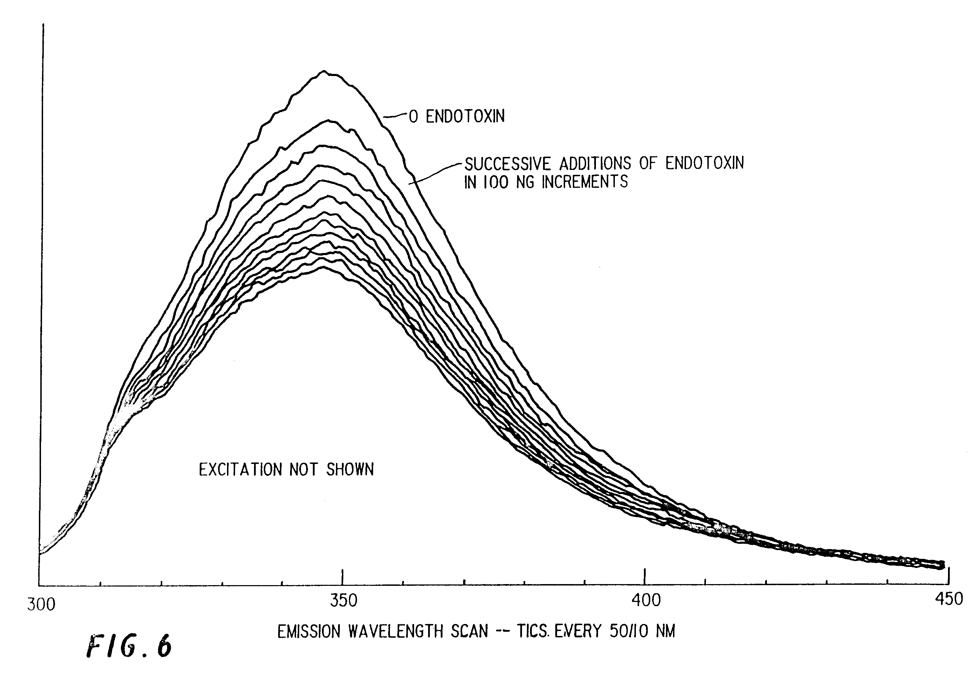

A known optical property of all proteins is the ability of some aromatic amino acids to fluoresce when excited at certain wavelengths. The present inventors looked specifically at tryptophan residues. These are excited at around 283 nm and fluoresce at around 350 nm. See FIGURE 6.

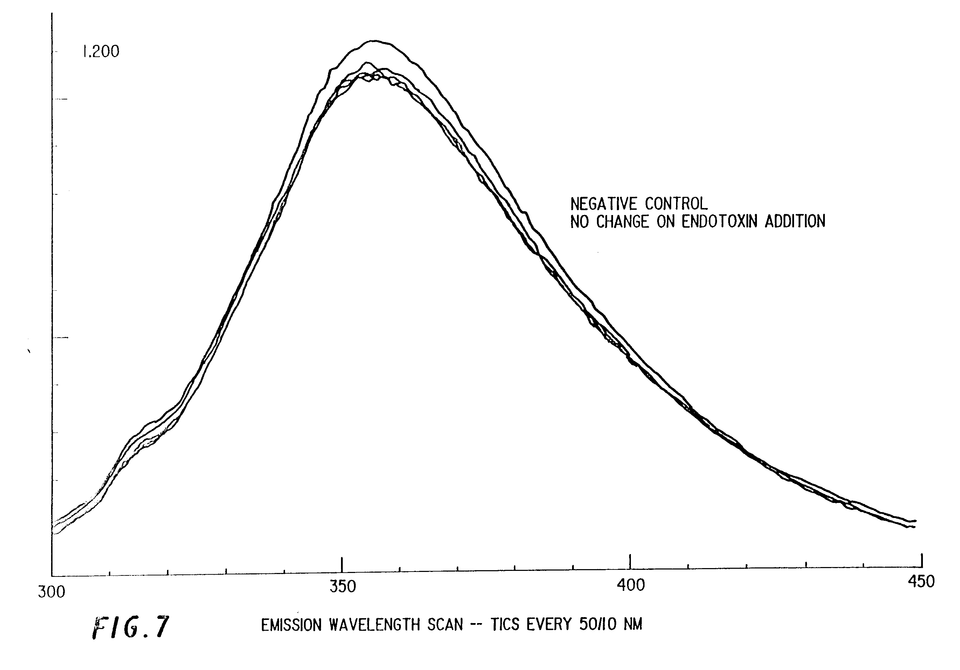

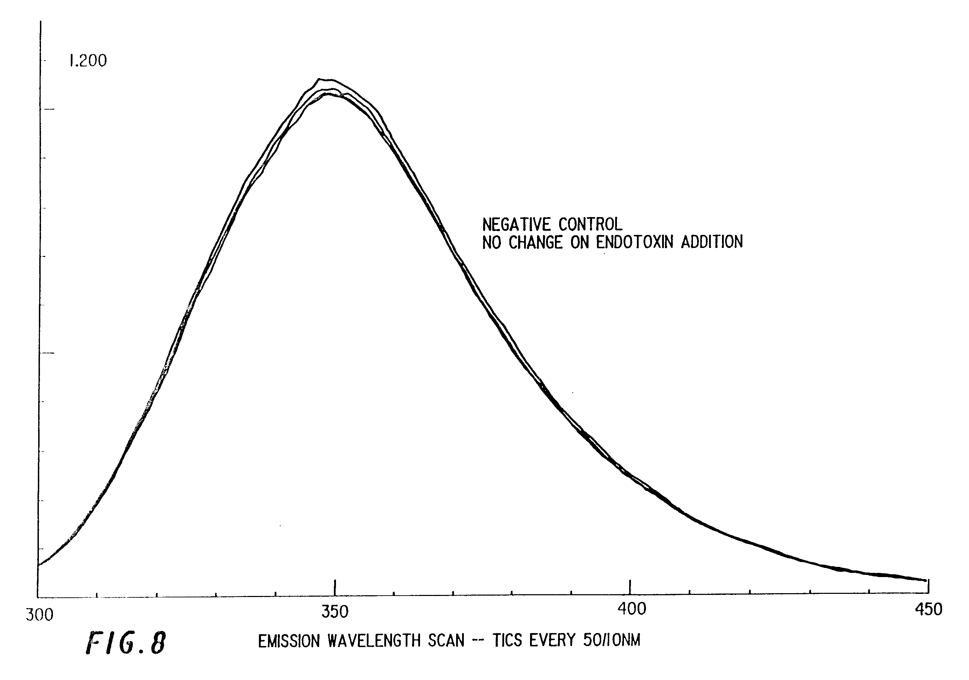

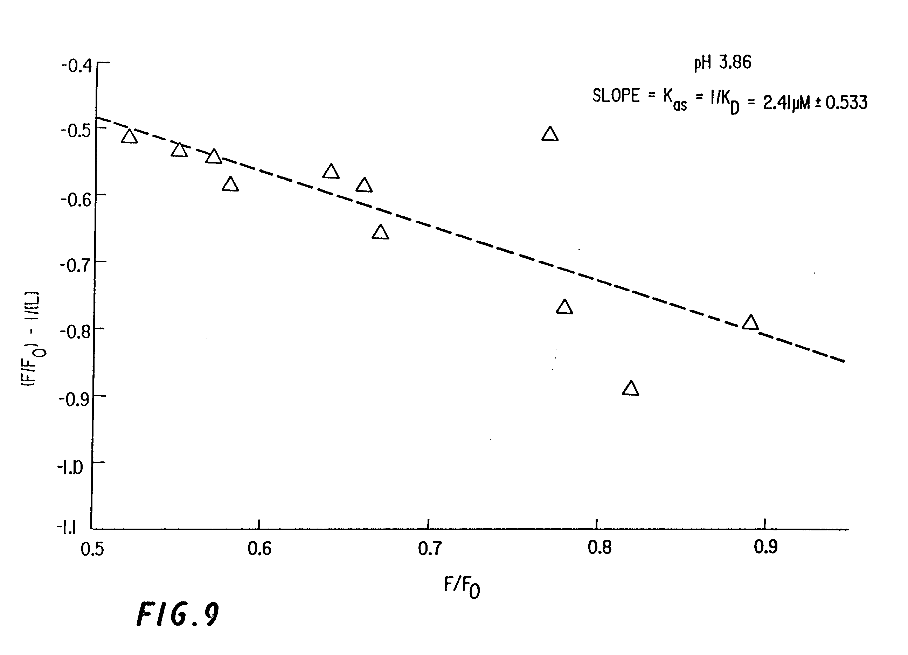

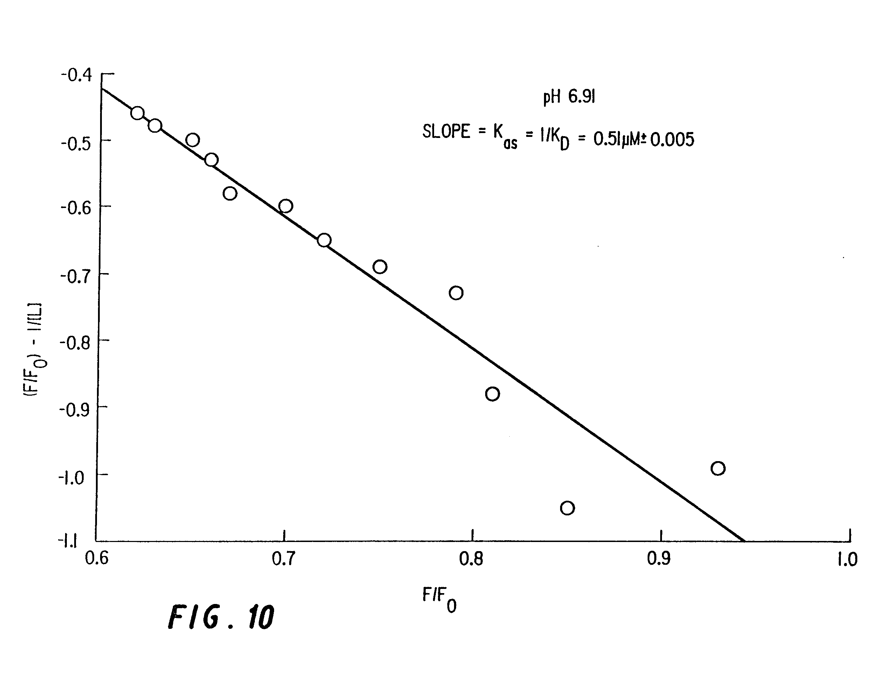

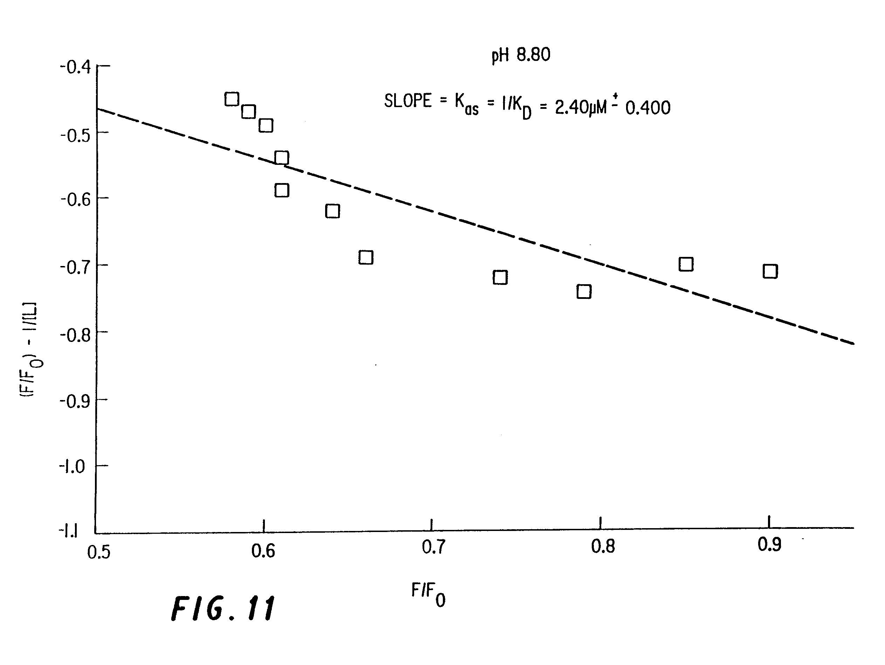

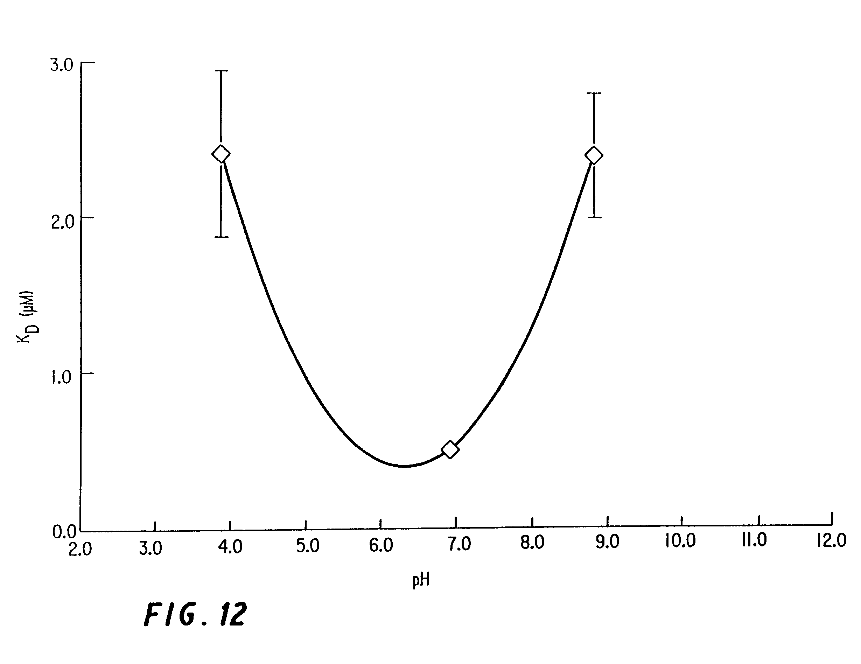

The inventors examined a fluorescence maxima at 350 nm of the endotoxin binding protein at various concentrations of endotoxin. There was a proportional quenching of that fluorescence, indicative of a tight association. See FIGURE 7. Other proteins as negative controls showed no such proportional change. See FIGURES 8 and 9. From this data, the inventors have been able to calculate the binding affinity constants between the present protein and endotoxin. See FIGURES 9-12.

In another preferred embodiment, the present invention relates to biosensors. Biosensors rely on a specific binding ligand immobilised on a solid (e.g. quartz) chip. Electrical potential between the two electrodes is measured.

The current method for measuring endotoxin is the Limulus amebocyte lysate assay. This involves a cascade of enzymes which are activated by endotoxin and result in the formation of a gel, analogous to a mammalian blood clot. Since multiple enzymes are involved, many substances interfere with the overall reaction, causing enhancement or inhibition. In addition, the key enzymes of the cascade are proteases so other proteolytic enzymes, such as trypsin, can cause gel formation. To eliminate these problems, samples containing interfering substances must be diluted beyond the point where they interfere. In many cases the dilution factor can be hundreds or thousands. The net effect is to decrease the sensitivity in measuring absolute endotoxin concentrations in those samples.

One of the promising potentials that a single endotoxin binding protein has is to eliminate the multiple interfering effects seen in the LAL assay. Even if the sensitivity were one tenth that of LAL, the lack of a need to dilute would compensate, making the sensitivity equivalent. Biosensors take advantage of specific binding affinities of such molecules. Antibodies are an example which have been used. Bound to the surface of a silicon chip, they bind to their immunogen and cause a change in the surface which is measured electrically. Depending on the type of sensor, capacitance, resistance or acoustic wave changes are measurable. Depending on the concentrations and volumes of the samples, the time required for each measurement can be from seconds to minutes.

The electronic component of the biosensor could measure voltage (potentiometric), current (amperometric), light, sound, temperature, or mass (piezoelectric). The biosensors of the present invention can be based on technology which is known, such as described in Biotechnology 5, 437-440 (1987), and Transducers 1987, the abstract entitled "Development of a Surface Acoustic Wave Biosensor", and "Recent Progress in Biosensor Development", Hall, E. A. H., Int. J. Biochem. 1988, 20(4), 357-62, each of which is incorporated herein by reference. Biosensor information is also contained in U.S. Patents 4,721,677, 4,680,268, H 0,000,201, and 4,614,714, each of which is incorporated herein by reference.

An actual device employing a biosensor would be similar to a flow cell. The sample would enter, be exposed to the reactive surface and a measurement made. Depending on the chemistry of binding, the bound ligand could be washed off to regenerate the surface or left on to measure a cumulative response. This may be adapted to an in-line device to monitor endotoxin contaminating events.

Due to the compact nature of the electronics, a portable field unit is feasible. This could be used on-site for checking water purity or process cleanliness during pharmaceutical manufacture, kidney dialysis unit pyrogen checks, or any other application where the conventional LAL is used now. Since endotoxin is a component of gram negative bacterial cell walls, binding of whole bacteria should be measurable. An extension of this technology should make possible remote sensors to monitor water quality in the environment.

The invention now being generally described, it will now be illustrated in greater detail by the following examples, which are presented herein for illustration only and are not intended to be limiting of the present invention, unless so indicated.

### Examples

### Example 1

Assay in of in vitro Inactivated Endotoxin by Pyrogen Testing in Rabbits:
E. coli endotoxin was prepared at 100 nanograms per ml. in Phosphate Buffered Saline (PBS). This was mixed with 200 micrograms of Limulus Endotoxin Binding Protein and incubated at 37 degrees Celsius for one hour. Control solutions were prepared of endotoxin (100 nanograms/ml) only and PBS only. Three vials of each of the three solutions were prepared containing 2 ml/vial. 1.5 ml of each solution was injected intravenously into individual 3 kilogram rabbits and their body temperatures measured continuously for six hours with data points recorded every 10 minutes. The final dose of endotoxin or inactivated endotoxin was 50 nanograms/kilogram.

In rabbits, a 5 nanograms/kilogram dose of endotoxin elicits a measurable fever response, showing a peak at one hour after injection. The present results showed the rabbits receiving only endotoxin at 50 nanograms/kilogram did indeed develop a body temperature elevated 1.64 (S.D. 0.236) degrees Celsius. Those rabbits receiving only PBS or endotoxin inactivated with Limulus Endotoxin Binding Protein maintained a normal body temperature.

### Example 2

Assay of In vivo Prophylactic Efficacy of Limulus Endotoxin Binding Protein:
Limulus Endotoxin Binding Protein is injected intravenously into rabbits at two dose levels, 5 micrograms/kilogram and 50 micrograms/kilogram. Control animals receive injections of PBS. Fifteen minutes later, all animals receive intravenous injections of 50 nanograms/kilogram E. coli endotoxin. Body temperature is monitored continuously over six hours and recorded every 10 minutes. The normal course of increase in temperature peaking at one hour after injection is seen in control animals preinjected with PBS only. Those animals receiving preinjection of endotoxin binding protein at 50 micrograms/kilogram showed an increase of 1.55 (S.D. 0.225) degrees Celsius. Animals receiving 5 micrograms/kilogram demonstrated temperature increases of 1.85 (S.D. 0.270).

### Example 3

Assay of In Vivo Therapeutic Efficacy of Limulus Endotoxin Binding Protein:
E. coli endotoxin is injected intravenously into 9 rabbits at a dose of 50 nanograms/kg. After 15 minutes, three were injected intravenously with 5 micrograms Limulus Endotoxin Binding Protein, three were injected intravenously with 50 micrograms Limulus Endotoxin Binding Protein and three received PBS. Volumes of all injections were 0.5 ml/kg. Body temperatures are monitored for 6 hours and data collected every 10 minutes. Animals receiving endotoxin and the PBS only, manifest the normal peak fever response one hour after toxin administration. Those animals receiving therapeutic injections of the Limulus protein exhibit a much reduced fever response proportional to the amount of protein which is administered.

### Example 4

Endotoxin Inactivating Potential of Limulus Endotoxin Binding Protein in Human Serum:
In order to assay the potential effectiveness of the endotoxin binding protein as a therapeutic or prophylactic agent for septic shock or related disorders in humans, the protein was tested for its ability to inactivate endotoxin in the presence of whole human serum. The assay was conducted in a 96-well tissue culture multiwell plates as described in detail in Novitsky, et al., J. Clin. Micro., 20:211-216 (1985). To each well were added in order 0.05 ml of serum only or serum with 25 micrograms/ml Limulus Endotoxin Binding Protein, 0.05 ml E. coli endotoxin solution. The plates were covered with Parafilm to prevent evaporation, agitated on a mechanical vibrating platform for 15 seconds and incubated at 37 degrees Celsius. Multiwell plates were then uncovered, and 0.1 ml of LAL was added to each well. The plates were subsequently handled and read as described for the LAL endotoxin assay. Serum with the added endotoxin binding protein was able to inactivate increasingly larger amounts of endotoxin (2,000 nanograms/ml) while serum alone was able to bind and/or inactivate only 80 nanograms/ml.

### Example 5

Universality of Endotoxin Type Inactivated by the Limulus Endotoxin Binding Protein.

In order to investigate the mechanism of the endotoxin binding phenomenon and determine the breadth of endotoxin types against which the binding protein is effective, endotoxin from several species of Gram negative bacteria as well as lipid A. were tested. These included endotoxin from Klebsiella pneumoniae, Serratia marcescens, Salmonella enteritidis, Eschericia coli 0113 wild type, E. coli rough mutant (J-5), Salmonella abortus equi, and Lipid A from S. minnesota Re 595.

The endotoxin binding protein was mixed with the endotoxins or lipid A at a ratio of 5:1 to 1,000:1 in the presence of 10 millimolar Tris buffer, pH 6-8. In all cases the measurable endotoxin after mixing was reduced 85% to 99.5%.

## Claims

1. An endotoxin binding protein having a molecular weight of 5,000 to 50,000 daltons and an isolectric pH greater than 10, isolated from horseshoe crab amebocytes for use as a prophylactic or active therapeutic substance.

2. The protein of claim 1 isolated from the horseshoe crab *Limulus polyphemus*.

3. A protein as claimed in claim 1 or claim 2, wherein said protein is obtainable by a process comprising the steps of:
(a) subjecting amebocytes obtained from a horseshoe crab to hypotonic shock to lyse said amebocytes, and obtaining cell debris from said lysed amebocytes;
(b) extracting the cell debris of step (a) with a solution containing a denaturant selected from the group consisting of urea and guanidine hydrochloride, to produce an extract;
(c) passing the extract of step (b) through a first ultrafiltration membrane having a molecular cutoff of from 20,000 to 50,000 daltons, to obtain a filtrate;
(d) concentrating the filtrate of step (c) by passing it through a second ultrafiltration membrane having a molecular cutoff of from 5,000 to 10,000 daltons, to produce a retentate;
(e) subjecting the retentate of step (d) to cation exchange chromatography at a pH of from about 5 to 6, using an elution buffer which comprises urea, and eluting a solution containing a peak of endotoxin binding activity;
(f) applying the solution containing said peak of endotoxin binding activity of step (e) to a reverse phase column, and adding a buffer to said reverse phase column, to obtain a solution containing purified endotoxin binding protein.

4. The protein of claim 3, wherein the hypotonic shock of step (a) is accomplished by treating said amebocytes with endotoxin-free distilled water at about 0° to 10°C.

5. The protein of claim 3 or claim 4, wherein the solution of step (b) is 6 molar urea or 6 molar guanidine hydrochloride.

6. A protein as claimed in any one of claims 3 to 5, wherein the extract of step (b) is crudely filtered with a filter aid selected from the group consisting of diatomaceous earth, cationic and anionic colloidal particle suspensions, prior to step (c).

7. A protein as claimed in any one of claims 3 to 6, wherein the ultrafiltration membranes of steps (c) and (d) are each composed of polysulfone.

8. The protein of claim 7, wherein the polysulfone membrane of step (c) has a molecular cutoff of 30,000 daltons.

9. The protein of claim 7, wherein the polysulfone membrane of step (d) has a molecular cutoff of 8,000 daltons.

10. A protein as claimed in any one of claims 3 to 9, wherein the cation exchange chromatography of step (e) is accomplished with Sepharose.

11. A protein as claimed in any one of claims 3 to 10, wherein the cation exchange chromatography of step (e) involves elution from a cation exchange column with a step gradient comprised of a solution of a salt selected from the group consisting of ammonium chloride, potassium chloride and sodium chloride.

12. A protein as claimed in any one of claims 3 to 10, wherein the cation exchange chromatography of step (e) involves elution from a cation exchange column with a buffer comprising 1 to 6 molar urea.

13. A protein as claimed in any one of claims 3 to 12, wherein the reverse phase column of step (f) comprises a resin having 4, 8 or 18 carbon atom chains.

14. A protein as claimed in any one of claims 3 to 13, eluted from the reverse phase column of step (f) with a step gradient of isopropanol and trifluoroacetic acid.

15. The protein of claim 14, wherein said step gradient has a concentration of trifluoroacetic acid ranging from 0.15 to 0.25%.

16. A medical preparation containing an endotoxin binding protein as defined in any of claims 1 to 15 and a pharmaceutically-inert excipient.

17. A substance for treating or preventing endotoxemia in a mammal consisting of the protein of any of claims 1 to 15 and a pharmaceutically-inert excipient.

18. The protein of any of claims 1 to 15 for use in a method for reducing endotoxin potency in a mammal.

19. The protein of any of claims 1 to 15 for use in a method for reducing endotoxin potency in a mammal by intravenous administration.

20. The use of a protein as defined in any one of claims 1 to 15 in the preparation of an agent for the treatment or prevention of endotoxemia or for reducing endotoxin potency in a mammal.

21. A biosensor device, which comprises the endotoxin binding protein of any of claims 1 to 15, immobilised on a solid support.

22. The biosensor device of claim 21, wherein said solid support is made of quartz or silicon.

23. The biosensor device of claim 21 or 22, wherein a measurement is obtained by a change in capacitance, resistance, or acoustic wave of said solid support.

## Patentansprüche

1. Endotoxin-Bindeprotein mit einem Molekulargewicht von 5.000 bis 50.000 Dalton und einem isoelektrischen pH von mehr als 10, isoliert aus Amöbocyten von Pfeilschwanzkrebsen, zur Verwendung als Prophylaktikum oder therapeutisch wirksame Substanz.

2. Protein nach Anspruch 1, isoliert aus dem Pfeilschwanzkrebs Limulus polyphemus.

3. Protein nach Anspruch 1 oder Anspruch 2, welches durch ein Verfahren erhältlich ist, das als Schritte umfaßt:
(a) Unterwerfen der von Pfeilschwanzkrebsen erhaltenen Amöbocyten einer hypotonen Schockbehandlung zur Lyse der Amöbocyten und Sammeln der Zelltrümmer der lysierten Amöbocyten;
(b) Extrahieren der Zelltrümmer aus Schritt (a) mit einer Lösung, die ein aus der aus Harnstoff und Guanidinhydrochlorid bestehenden Gruppe ausgewähltes Denaturierungsmittel enthält, zur Erzeugung eines Extrakts;
(c) Passieren des Extrakts aus Schritt (b) durch eine erste Ultrafiltrationsmembran mit einem molekularen Schnitt bei 20.000 bis 50.000 Dalton zwecks Erzielung eines Filtrats;
(d) Konzentrieren des Filtrats aus Schritt (c) durch Passieren desselben durch eine zweite Ultrafiltrationsmembran mit einem molekularen Schnitt bei 5.000 bis 10.000 Dalton, zur Erzeugung eines Retentats;
(e) Unterwerfen des Retentats aus Schritt (d) einer Kationenaustauschchromatographie bei einem pH von etwa 5 bis 6 unter Verwendung eines Harnstoff enthaltenden Elutionspuffers und Eluieren einer Lösung mit einem Peak mit Endotoxin-Bindeaktivität;
(f) Aufbringen der diesen Peak mit Endotoxin-Bindeaktivität aus Schritt (e) enthaltenen Lösung auf eine Umkehrphasensäule und Zugabe eines Puffers zu der Umkehrphasensäule zwecks Erhalt einer Lösung, die gereinigtes Endotoxin-Bindeprotein enthält.

4. Protein nach Anspruch 3, worin die hypotone Schockbehandlung in Schritt (a) durch Behandlung der Amöbocyten mit Endoxin-freiem destilliertem Wasser bei etwa 0 bis 10°C bewirkt wird.

5. Protein nach Anspruch 3 oder Anspruch 4, worin die Lösung aus Schritt (b) 6-molarer Harnstoff oder 6-molares Guanidinhydrochlorid ist.

6. Protein nach einem der Ansprüche 3 bis 5, worin der Extrakt aus Schritt (b) vor Schritt (c) grob mit einer Filtrierhilfe, ausgewählt aus der aus Diatomeenerde, kationischen und anionischen kolloidalen Teilchensuspensionen bestehenden Gruppe, filtriert wird.

7. Verfahren nach einem der Ansprüche 3 bis 6, worin die Ultrafiltrationsmembrane der Schritte (c) und (d) jeweils aus Polysulfon zusammengesetzt sind.

8. Protein nach Anspruch 7, worin die Polysulfonmembran aus Schritt (c) einen molekularen Schnitt bei 30.000 Dalton setzt.

9. Protein nach Anspruch 7, worin die Polysulfonmembran aus Schritt (d) einen molekularen Schnitt bei 8.000 Dalton setzt.

10. Protein nach einem der Ansprüche 3 bis 9, worin die Kationenaustauschchromatographie von Schritt (e) mit Sepharose bewirkt wird.

11. Protein nach einem der Ansprüche 3 bis 10, worin die Kationenaustauschchromatographie von Schritt (e) die Elution von einer Kationenaustauschsäule mit einem stufenweisen Gradienten beinhaltet und eine Lösung eines aus der aus Ammoniumchlorid, Kaliumchlorid und Natriumchlorid bestehenden Gruppe ausgewählten Salzes einschließt.

12. Protein nach einem der Ansprüche 3 bis 10, worin die Kationenaustauschchromatographie von Schritt (e) die Elution von einer Kationenaustauschsäule mit einem 1- bis 6-molaren Harnstoff enthaltenen Puffer beinhaltet.

13. Protein nach einem der Ansprüche 3 bis 12, worin die Umkehrphasensäule von Schritt (f) ein Harz mit Ketten mit 4, 8 oder 18 Kohlenstoffatomen umfaßt.

14. Protein nach einem der Ansprüche 3 bis 13, eluiert von der Umkehrphasensäule von Schritt (f) mit stufenweisem Gradienten mit Isopropanol und Trifluoressigsäure.

15. Protein nach Anspruch 14, worin der stufenweise Gradient eine Trifluoressigsäurekonzentration im Bereich von 0,15 bis 0,25 % aufweist.

16. Medizinische Zubereitung mit einem Endotoxin-Bindeprotein, wie in einem der Ansprüche 1 bis 15 definiert, sowie einem pharmazeutisch inerten Exzipienten.

17. Substanz zur Behandlung oder Vorbeugung von Endotoxämie in einem Säugetier, die aus dem Protein nach einem der Ansprüche 1 bis 15 und einem pharmazeutisch inerten Exzipienten besteht.

18. Protein nach einem der Ansprüche 1 bis 15 zur Verwendung in einem Verfahren zur Reduzierung der Endotoxinwirksamkeit in einem Säugetier.

19. Protein nach einem der Ansprüche 1 bis 15 zur Verwendung in einem Verfahren zur Reduzierung der Endotoxinwirksamkeit in einem Säugetier durch intravenöse Verabreichung.

20. Verwendung eines Proteins, wie in einem der Ansprüche 1 bis 15 definiert, zur Zubereitung eines Mittels zur Behandlung oder Vorbeugung von Endotoxämie oder zur Reduzierung der Endotoxinwirksamkeit in einem Säugetier.

21. Biosensorvorrichtung, die das Endotoxin-Bindeprotein nach einem der Ansprüche 1 bis 15 immobilisiert auf einem festen Träger enthält.

22. Biosensorvorrichtung nach Anspruch 21, worin der feste Träger aus Quarz oder Silikon besteht.

23. Biosensorvorrichtung nach Anspruch 21 oder 22, worin ein Meßwert über eine Änderung in der Kapazität, im Widerstand oder der akkustischen Welle des festen Trägers erhalten wird.

## Revendications

1. Protéine de liaison aux endotoxines, ayant une masse moléculaire de 5 000 à 50 000 daltons et un pH isoélectrique supérieur à 10, isolée à partir d'amibocytes de limule, pour une utilisation en tant que substance prophylactique ou thérapeutique active.

2. Protéine selon la revendication 1, isolée à partir de la limule *Limulus polyphemus*.

3. Protéine selon la revendication 1 ou la revendication 2, celle-ci pouvant être obtenue par un procédé comprenant les étapes consistant à:
(a) soumettre des amibocytes obtenus à partir d'une limule à un choc hypotonique pour lyser lesdits amibocytes, et obtenir des débris cellulaires à partir desdits amibocytes lysés ;
(b) extraire les débris cellulaires de l'étape (a) par une solution contenant un dénaturant choisi dans le groupe constitué par l'urée et le chlorhydrate de guanidine, afin de produire un extrait ;
(c) faire passer l'extrait de l'étape (b) à travers une première membrane d'ultrafiltration ayant un seuil de coupure des masses moléculaires de 20 000 à 50 000 daltons, afin d'obtenir un filtrat ;
(d) concentrer le filtrat de l'étape (c) en le faisant passer à travers une seconde membrane d'ultrafiltration ayant un seuil de coupure des masses moléculaires de 5 000 à 10 000 daltons, afin de produire un rétentat ;
(e) soumettre le rétentat de l'étape (d) à une chromatographie d'échange de cations à un pH d'environ 5 à 6, à l'aide d'un tampon d'élution qui comprend de l'urée, et éluer une solution contenant un pic d'activité de liaison aux endotoxines ;
(f) appliquer la solution contenant ledit pic d'activité de liaison aux endotoxines de l'étape (e) sur une colonne phase inverse, et ajouter un tampon à ladite colonne phase inverse, afin d'obtenir une solution contenant une protéine de liaison aux endotoxines purifiée.

4. Protéine selon la revendication 3, pour laquelle le choc hypotonique de l'étape (a) est réalisé par traitement desdits amibocytes par de l'eau distillée exempte d'endotoxines à environ 0 à 10°C.

5. Protéine selon la revendication 3 ou la revendication 4, pour laquelle la solution de l'étape (b) est de l'urée 6 fois molaire ou du chlorhydrate de guanidine 6 fois molaire.

6. Protéine selon l'une des revendications 3 à 5, pour laquelle l'extrait de l'étape (b) est grossièrement filtré avec un adjuvant de filtration choisi dans le groupe constitué par la terre à diatomées, les suspensions de particules colloïdales cationiques et anioniques, avant l'étape (c).

7. Protéine selon l'une des revendications 3 à 6, pour laquelle les membranes d'ultrafiltration des étapes (c) et (d) sont chacune composées de polysulfone.

8. Protéine selon la revendication 7, pour laquelle la membrane de polysulfone de l'étape (c) a un seuil de coupure des masses moléculaires de 30 000 daltons.

9. Protéine selon la revendication 7, pour laquelle la membrane de polysulfone de l'étape (d) a un seuil de coupure des masses moléculaires de 8 000 daltons.

10. Protéine selon l'une des revendications 3 à 9, pour laquelle la chromatographie d'échange de cations de l'étape (e) est réalisée avec du Sepharose.

11. Protéine selon l'une des revendications 3 à 10, pour laquelle la chromatographie d'échange de cations de l'étape (e) met en jeu une élution à partir d'une colonne d'échange de cations avec un gradient étagé composé d'une solution d'un sel choisi dans le groupe constitué par le chlorure d'ammonium, le chlorure de potassium et le chlorure de sodium.

12. Protéine selon l'une des revendications 3 à 10, pour laquelle la chromatographie d'échange de cations de l'étape (e) met en jeu une élution à partir d'une colonne d'échange de cations avec un tampon comprenant de l'urée 1 à 6 fois molaire.

13. Protéine selon l'une des revendications 3 à 12, pour laquelle la colonne phase inverse de l'étape (f) comprend une résine ayant des chaînes à 4, 8 ou 18 atomes de carbone.

14. Protéine selon l'une des revendications 3 à 13, éluée à partir de la colonne phase inverse de l'étape (f) avec un gradient étagé d'isopropanol et d'acide trifluoroacétique.

15. Protéine selon la revendication 14, pour laquelle ledit gradient étagé a une concentration en acide trifluoroacétique se situant dans une plage allant de 0,15 à 0,25%.

16. Préparation médicale contenant une protéine de liaison aux endotoxines telle que définie à l'une des revendications 1 à 15 et un excipient pharmaceutiquement inerte.

17. Substance pour le traitement ou la prévention de l'endotoxémie chez un mammifère, consistant en la protéine telle que définie à l'une des revendications 1 à 15 et en un excipient pharmaceutiquement inerte.

18. Protéine selon l'une des revendications 1 à 15, pour une utilisation dans une méthode de réduction de la puissance des endotoxines chez un mammifère.

19. Protéine selon l'une des revendications 1 à 15, pour une utilisation dans une méthode de réduction de la puissance des endotoxines chez un mammifère par administration intraveineuse.

20. Utilisation d'une protéine telle que définie à l'une des revendications 1 à 15, dans la préparation d'un agent pour le traitement ou la prévention de l'endotoxémie ou pour la réduction de la puissance des endotoxines chez un mammifère.

21. Dispositif biodétecteur, qui comprend la protéine de liaison aux endotoxines telles que définie à l'une des revendications 1 à 15, immobilisée sur un support solide.

22. Dispositif biodétecteur selon la revendication 21, dans lequel ledit support solide est fait de quartz ou de silicium.

23. Dispositif biodétecteur selon la revendication 21 ou 22, dans lequel une mesure est obtenue par un changement dans la capacitance, la résistance ou l'onde acoustique dudit support solide.
